(19) Europäisches Patentamt
European Patent Office
Office européen des brevets

(11) Veröffentlichungsnummer: **0 229 630**
**A2**

# (12) EUROPÄISCHE PATENTANMELDUNG

(21) Anmeldenummer: 87100110.3

(22) Anmeldetag: 08.01.87

(51) Int. Cl.[4]: **C 07 D 211/22**
A 01 N 47/36

(30) Priorität: 16.01.86 DE 3601048

(43) Veröffentlichungstag der Anmeldung: 22.07.87 Patentblatt 87/30

(84) Benannte Vertragsstaaten: AT BE CH DE ES FR GB GR IT LI LU NL SE

(71) Anmelder: HOECHST AKTIENGESELLSCHAFT
Postfach 80 03 20
D-6230 Frankfurt am Main 80 (DE)

(72) Erfinder: Liebl, Rainer, Dr.
An der Weinleite 5b
D-8901 Todtenweis (DE)

Frey, Michael, Dr.
Kapellenstrasse 17
D-8906 Gersthofen (DE)

Mildenberger, Hilmar, Dr.
Fasanenstrasse 24
D-6233 Klekheim (Taunus) (DE)

Bauer, Klaus, Dr.
Kolpingstrasse 7
D-6054 Rodgau (DE)

Bieringer, Hermann, Dr.
Eichenweg 26
D-6239 Eppstein/Taunus (DE)

## (54) 1,2-Disubstituierte Piperidine, Verfahren zu ihrer Herstellung sowie ihre Verwendung im Pflanzenschutz.

(57) 1,2-Disubstituierte Piperidine der allgemeinen Formel I

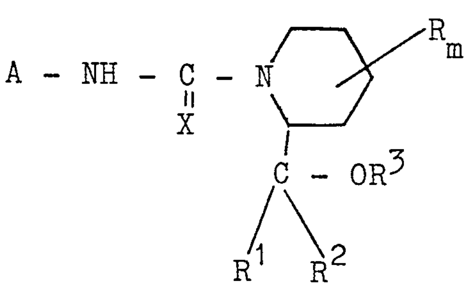

I,

worin X = Sauerstoff oder Schwefel; R = Alkyl, $R^1$, $R^2$ = Wasserstoff, Alkyl, oder Benzyl, $R^3$ = Wasserstoff, (subst.)Alkyl, (subst.) Benzyl oder einen Rest der Formeln

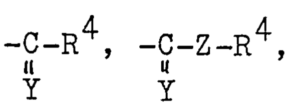

, $-S(O)_n-R^4$ oder

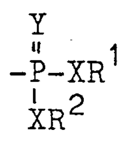

, Y, X = S oder O;

Z = S, O oder $NR^1$; $R^4$ = Wasserstoff, (subst.)Alkyl, (subst.) Phenyl, (subst.) Naphthyl oder (subst.) Pyridyl, A = (subst.) Phenyl oder (subst.) Naphthyl und m = 0, 1 oder 2 bedeuten, besitzen vorteilhafte herbizide Eigenschaften.

EP 0 229 630 A2

Bundesdruckerei Berlin

**Beschreibung**

1,2-Disubstituierte Piperidine, Verfahren zu ihrer Herstellung sowie ihre Verwendung im Pflanzenschutz

Cyclische piperidinhaltige Harnstoffverbindungen mit herbizider Wirksamkeit sind aus Environmental Quality and Safety Suppl. (Pesticides) Bd. 3 S. 696 (1975) bekannt. Diese besitzen jedoch unzureichende Herbizidwirkungen. Gegenstand der vorliegenden Erfindung sind neue 1,2-disubstituierte Piperidine der allgemeinen Formel I

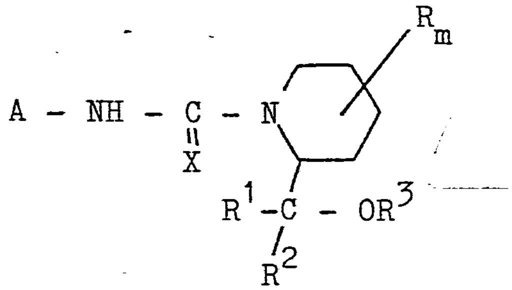

I,

worin
X Sauerstoff oder Schwefel;
R $(C_1-C_4)$Alkyl
$R^1$, $R^2$ unabhängig voneinander Wasserstoff, $(C_1-C_4)$Alkyl, Phenyl oder Benzyl, die beide bis zu dreifach durch $(C_1-C_4)$Alkyl, Halogen, $CF_3$, $NO_2$ oder CN substituiert sein können,
$R^3$ Wasserstoff, $(C_1-C_4)$Alkyl, das unsubstituiert oder bis zu dreifach substituiert ist durch Halogen, $(C_3-C_6)$Cycloalkyl, $(C_3-C_4)$Alkenyl, $(C_5-C_6)$Cycloalkenyl, $(C_3-C_4)$Alkinyl, $(C_1-C_4)$Alkoxy-$(C_1-C_4)$-alkyl oder $(C_1-C_4)$Alkoxycarbonyl-$(C_1-C_4)$alkyl; Phenyl oder Benzyl, die beide unsubstituiert oder bis zu dreifach substituiert sind durch Halogen, $(C_1-C_4)$Alkyl, $NO_2$, $CF_3$, $(C_1-C_4)$Alkoxy oder $(C_1-C_4)$Alkoxycarbonyl; oder einen Rest der Formeln

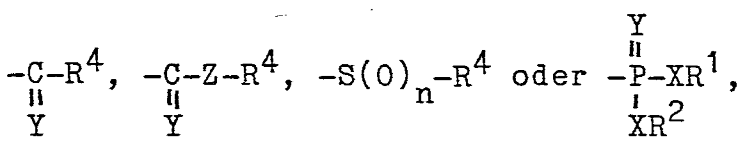

Y Sauerstoff oder Schwefel,
Z Sauerstoff, Schwefel oder $-NR^1$,
$R^4$ Wasserstoff, $(C_1-C_8)$Alkyl, das unsubstituiert oder bis zu dreifach substituiert ist durch Halogen, Phenyl oder Benzyl, die beide unsubstituiert oder bis zu dreifach am Aromaten substituiert sind durch Halogen, $(C_1-C_4)$Alkyl, $(C_1-C_4)$Alkoxy, $(C_1-C_4)$Alkoxycarbonyl, $NO_2$, $CF_3$ oder CN,
m 0, 1 oder 2,
n 1 oder 2,
A Phenyl oder Naphthyl, die beide unsubstituiert oder bis zu vierfach substituiert sind durch Halogen, $(C_1-C_4)$Alkyl, $(C_1-C_4)$Halogenalkyl, $(C_1-C_4)$Alkoxy, $(C_1-C_4)$Halogenalkoxy, $(C_3-C_6)$Cycloalkoxy, $(C_2-C_4)$Alkenyloxy, $(C_2-C_4)$Halogenalkenyloxy, $(C_5-C_6)$Cycloalkenyloxy, $(C_3-C_4)$Alkinyloxy, $(C_3-C_4)$Halogenalkinyloxy, $(C_1-C_4)$Alkoxy$(C_1-C_4)$alkoxy, $(C_1-C_4)$Alkoxycarbonyl, $(C_1-C_4)$Alkoxycarbonyl$(C_1-C_4)$alkoxy, $(C_1-C_4)$Alkoxycarbonyl-$(C_1-C_4)$alkoxycarbonyl, $(C_1-C_4)$Alkylthio, $(C_1-C_4)$Alkylsulfinyl, $(C_1-C_4)$Alkylsulfonyl, $(C_1-C_4)$Alkoxycarbonyl$(C_1-C_4)$alkylthio, Phenoxy, Benzyloxy, Phenoxymethyl, wobei die drei letztgenannten Reste im Phenylring bis zu zweifach durch Halogen, $CF_3$ oder $(C_1-C_4)$Alkyl substituiert sein können, $NO_2$, $CF_3$, CN oder Pyridyl, das unsubstituiert oder bis zu dreifach durch Halogen, $(C_1-C_4)$Alkyl, $NO_2$, $CF_3$ oder $(C_1-C_4)$- Alkoxy substituiert ist,
bedeuten.

Bevorzugte Verbindungen sind solche, bei denen R = $(C_1-C_4)$Alkyl,
$R^1$ und $R^2$ unabhängig voneinander Wasserstoff. $(C_1-C_4)$Alkyl $R^3$ Wasserstoff, $(C_1-C_4)$Alkyl. 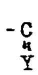 $-R^4$ oder

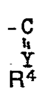 $-Z-R^4$,
$R^4$ $(C_1-C_4)$Alkyl, Phenyl, das unsubstituiert oder bis zu zweifach substituiert ist durch Halogen, $(C_1-C_4)$Alkyl oder $CF_3$,
A Phenyl, das bis zu dreifach durch Halogen, $(C_1-C_4)$Alkoxy, $(C_1-C_4)$Halogenalkoxy, $(C_1-C_4)$Alkylthio, $(C_1-C_4)$Alkylsulfonyl, Propargyloxy, $(C_1-C_4)$Alkoxycarbonyl oder $(C_1-C_4)$Alkyloxycarbonyl$(C_1-C_4)$alkoxy, substituiert ist, und
m 0 oder 1
bedeuten.

Besonders bevorzugt sind Verbindungen der Formel I, worin R = $(C_1-C_4)$Alkyl; $R^1$, $R^2$ = H oder

$(C_1-C_4)$Alkyl, $R^3 =$ H, $(C_1-C_4)$Alkyl oder $-CO(C_1-C_4$-alkyl) und A = ein Phenylrest, der ein- bis dreifach durch F, Cl, Br, $(C_1-C_4)$Alkoxy, $(C_1-C_4)$Alkoxycarbonyl-$(C_1-C_4)$alkoxy oder Propargyloxy substituiert ist, wobei die Reste insbesondere in 2-, 4- oder 5-Stellung des Phenylrestes orientiert sind, bedeuten.

Die Bezeichnung Halogenalkyl, Halogenalkoxy, Halogenalkenyl oder Halogenalkinyl bedeutet jeweils einen Alkyl-, Alkoxy-, Alkenyl- oder Alkinyl-Rest, der ein- oder mehrfach durch Halogen, insbesondere F, Cl oder Br, substituiert ist. Unter Halogenalkyl ist beispielsweise $CF_3$, $C_2F_5$, $CF_2Cl$, $CF_2CHF_2$, $CH_2CF_3$, $CF_2CHFCl$, $CF_2CHFCF_3$ oder $CF_2CHFBr$ zu verstehen. Haloalkoxy umfaßt beispielsweise die Reste $OCF_3$, $OCF_2CHF_2$, $OCF_2CHFCl$, $OCF_2CHCl_2$, $OCF_2CHFBr$ oder $OCF_2-CHF-CF_3$.

Die Verbindungen der Formel I besitzen im Piperidin-Ring ein oder mehrere Asymmetriezentren und können daher in Form von reinen optischen Isomeren, Diastereomeren oder deren Gemische vorliegen. Formel I umfaßt alle diese Isomeren bzw. Isomerengemische.

Ein weiterer Gegenstand der vorliegenden Erfindung sind Verfahren zur Herstellung von Verbindungen der allgemeinen Formel I, dadurch gekennzeichnet, daß man

a) eine Verbindung der Formel II mit einem Piperidin-Derivat der Formel III

$$A - N = C = X \quad \text{(II)}$$

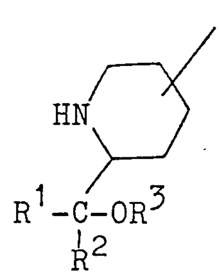

(III)

b) oder ein Amin der Formel IV mit einer Verbindung der Formel V

$A-NH_2$ (IV) +

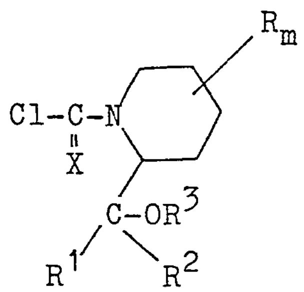

(V)

in Gegenwart einer Base umsetzt und die unter a) oder b) erhaltenen Verbindungen der Formel I für $R^3 =$ H gegebenenfalls O-alkyliert oder O-acyliert.

Die Verfahrensvariante a) wird in einem organischen Lösungsmittel wie z.B. Ether, Tetrahydrofuran, Toluol, Aceton, Acetonitril, Methylenchlorid oder Chloroform durchgeführt. Bei Variante b) kann als Base eine anorganische Base wie ein Alkalimetallhydroxid oder -carbonat oder eine organische Base wie ein organisches Amin, z.B. Pyridin oder Triethylamin eingesetzt werden. Auch diese Reaktion wird in einem unter den Reaktionsbedingungen inerten organischen Lösungsmittel durchgeführt.

Die Isocyanate bzw. Isothiocyanate der Formel II sowie die Amine der Formel IV sind bekannt oder lassen sich nach dem Fachmann geläufigen Methoden herstellen, s. Houben Weyl, Methoden der Organischen Chemie, Bd. VIII S. 120, Georg Thieme Verlag Stuttgart; Houben Weyl, Band IX S. 875, 869. Die Piperidine der Formel (III) sowie die Carbamoylchloride der Formel (V) sind teilweise bekannt oder lassen sich aus 2-Hydroxyalkylpiperidinen nach bekannten Synthesemethoden herstellen, vgl. J. Am. Chem. Soc. 77 (1955), 29, J. Am. Chem. Soc. 69 (1947), 3039, J. Am. Chem. Soc. 61 (1939), 638; Soc. 1949, 2095.

Die Alkylierung oder Acylierung der Verbindungen der Formel I mit $R^3 =$ H erfolgt nach dem Fachmann geläufigen Methoden s. beispielsweise Organikum, 15. Auflage S. 253, 505, VEB Verlag, Berlin.

Die erfindungsgemäßen Verbindungen der Formel I weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden.

Werden die erfindungsgemäßen Verbindungen vor dem Keimen auf die Erdoberfläche appliziert, so wird entweder das Auflaufen der Unkrautkeimlinge vollständig verhindert, oder die Unkräuter wachsen bis zum Keimblattstadium heran, stellen jedoch dann ihr Wachstum ein und sterben schließlich nach Ablauf von drei bis fünf Wochen vollkommen ab. Bei Applikation der Wirkstoffe auf die grünen Pflanzenteile im Nachauflaufverfahren tritt ebenfalls sehr rasch nach der Behandlung ein drastischer Wachstumsstop ein, und

die Unkrautpflanzen bleiben in dem zum Applikationszeitpunkt vorhandenen Wachstumsstadium stehen oder sterben nach einer gewissen Zeit mehr oder weniger schnell ganz ab, so daß auf diese Weise eine für die Kulturpflanzen schädliche Unkrautkonkurrenz sehr früh und nachhaltig durch den Einsatz der neuen erfindungsgemäßen Mittel beseitigt werden kann.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z. B. Weizen, Gerste, Roggen, Reis, Mais, ~~Zuckerrübe,~~ Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüber hinaus weisen die erfindungsgemäßen Verbindungen wachstumsregulatorische Eigenschaften bei Kulturpflanzen auf. Sie greifen regulierend in den pflanzeneigenen Stoffwechsel ein und können damit zur Ernteerleichterung wie z. B. durch Auslösen von Desikkation, Abszission und Wuchsstauchung eingesetzt werden. Desweiteren eignen sie sich auch zur generellen Steuerung und Hemmung von unerwünschtevegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Mittel können als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Spritzpulver sind in Wasser gleichmäßig dispergierbare Präparate, die neben dem Wirkstoff außer gegebenenfalls einem Verdünnungs- oder Inertstoff noch Netzmittel, z. B. polyoxethylierte Alkylphenole, polyoxethylierte Fettalkohole, Alkyl- oder Alkylphenylsulfonate und Dispergiermittel, z. B. ligninsulfonsaures Natrium, 2,2′-dinaphthylmethan-6,6′-disulfonsaures Natrium, dibutylnaphthalinsulfonsaures Natrium oder auch oleoylmethyltaurinsaures Natrium enthalten. Die Herstellung erfolgt in üblicher Weise, z. B. durch Mahlen und Vermischen der Komponenten.

Emulgierbare Konzentrate können z. B. durch Auflösen des Wirkstoffes in einem inerten organischen Lösungsmittel, z. B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendet werden: Alkylarylsulfonsaure Calciumsalze wie Ca=dodecylbenzolsulfonat oder nichtionische Emulgatoren wie Fettsäurepolyglykolester, Alky-arylpolyglykolether, Fettalkoholpolyglykolether, Propylenoxid-Ethylenoxid-Kondensationsprodukte, Fettalkohol-Propylenoxid-Ethylenoxid-Koncensationsprodukte, Alkylpolyglykolether, Sorbitanfettsäureester, Polyoxethylensorbitanfettsäureester oder Polyoxethylensorbitester.

Stäubemittel kann man durch Vermahlen des Wirkstoffes mit feinverteilten, festen Stoffen z. B. Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentrationen mittels Bindemitteln, z. B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise, gewünschtenfalls in Mischung mit Düngemitteln, granuliert werden.

In Spritzpulvern beträgt die Wirkstoffkonzentration z. B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-% an Wirkstoff, versprüh bare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe unsw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll-oder Trägerstoffe.

Zur Anwendung werden die in handelsüblicher Form vorliegenden Konzentrate gegebenenfalls in üblicher Weise verdünnt, z. B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u. a. variiert die erforderliche Aufwandmenge. Sie kann innerhalb weiter Granzen schwanken, z. B. zwischen 0,005 und 10,0 kg/ha oder mehr Aktivsubstanz, vorzugsweise liegt sie jedoch zwischen 0.01 und 5 kg/ha.

Auch Mischungen oder Mischformulierungen mit anderen Wirkstoffen, wie z. B. Insektiziden, Akariziden, Herbiziden, Düngemitteln, Wachstumsregulatoren oder Fungiziden sind gegebenenfalls möglich.

Die Erfindung wird durch nachstehende Beispiele näher erläutert.

Formulierungsbeispiele

A. Ein Stäubemittel wird erhalten, indem man 10 Gewichtsteile Wirkstoff und 90 Gewichtsteile Talkum oder Inertstoff mischt und in einer Schlagmühle zerkleinert.

B. Ein in Wasser leicht dispergierbares, benetzbares Pulver wird erhalten, indem man 25 Gewichtsteile

Wirkstoff, 64 Gewichtsteile kaolinhaltigen Quarz als Inertstoff, 10 Gewichtsteile ligninsulfonsaures Kalium und 1 Gewichtsteil oleoylmethyltaurinsaures Natrium als Netz- und Dispergiermittel mischt und in einer Stiftmühle mahlt.

C. Ein in Wasser leicht dispergierbares Dispersionskonzentrat wird erhalten, indem man 20 Gewichtsteile Wirkstoff mit 6 Gewichtsteilen Alkylphenolpolyglykolether (Triton X 207), 3 Gewichtsteilen Isotridecanolpolyglykolether (8 AeO) und 71 Gewichtsteilen paraffinischem Mineralöl (Siedebereich z. B. ca. 255 bis über 377 °C) mischt und in einer Reibkugelmühle auf eine Feinneit von unter 5 Mikron vermahlt.

D. Ein emulgierbares Konzentrat wird erhalten aus 15 Gewichtsteilen Wirkstoff, 75 Gewichtsteilen Cyclohexanon als Lösungsmittel und 10 Gewichtsteilen oxethyliertes Nonylphenol (10 AeO) als Emulgator.

Chemische Beispiele

Beispiel 1

1-[N-(4-Chlor-2-fluor-5-methoxy-phenyl)-carbamoyl]-2-hydroxy-methyl-piperidin

Man löst 10,1 g (0,05 mol) 4-Chlor-2-fluor-5-methoxy-phenylisocyanat in 50 ml Toluol und tropft bei RT (RT= Raumtemperatur) 5,8g (0,05 mol) 2-Hydroxymethyl-piperidin, gelöst in 20 ml Toluol, zu. Man rührt 2 h bei 20 - 25 °C, kühlt im Eisbad und saugt den ausgefallenen Niederschlag ab. Nach dem Trocknen erhält man 12,6 g (79 % d. Th.) 1-[N-(4-Chlor-2-fluor-5-methoxy-phenyl)-carbamoyl]-2-hydroxymethyl-piperidin in Form farbloser Kristalle mit Schmp. 176 - 179 °C.

Beispiel 2

2-Hydroxymethyl-1-{N-[3-methyl-4-(1,1,2,2-tetrafluorethoxy)-phenyl]-carbamoyl}-piperidin

Man löst 11,5 g (0,10 mol) 2-Hydroxymethylpiperidin in 100 ml Ether und tropft bei 20 °C 24,9 g (0,10 mol) 3-Methyl-4-(1,1,2,2-tetrafluorethoxy)-phenylisocyanat, gelöst in 70 ml Ether, zu. Man rührt 2 h bei 20 °C, kühlt im Eisbad und saugt den ausgefallenen Niederschlag ab. Nach dem Trocknen erhält man 32,1 g (88 % d. Th.) 2-Hydroxymethyl-1-{N-[3-methyl-4-(1,1,2,-tetrafluorethoxy)-phenyl]-carbamoyl}-piperidin in Form farbloser Kristalle mit Schmp. 122 - 126 °C.

Beispiel 3

1-[N-(4-Chlor-2-fluor-5-methoxy-phenyl)-thiocarbamoyl]-2-hydroxymethyl-piperidin

Man löst 11,5 g (0,10 mol) 2-Hydroxymethylpiperidin in 100 ml Ether und tropft bei RT 21,8 g (0,10 mol) 4-Chlor-2-fluor-5-methoxy-phenyl-isothiocyanat zu. Man rührt 2 h bei 30 °C, wäscht zweimal mit je 100 ml Wasser, trocknet über Natriumsulfat und zieht das Lösungsmittel ab. Man erhält 32,6 g (98 % d. Th.) 1-[N-(4-Chlor-2-fluor-5-methoxy-phenyl)-thiocarbamoyl]-2-hydroxymethyl-piperidin in Form eines hellgelben zähen Öles.

Beispiel 4

2-(N-Methyl-carbamoyloxymethyl)-1-{N-[3-methyl-4-(1.1.2.2-tetrafluorethoxy)-phenyl]-carbamoyl}-piperidin

Man löst 18,2 g (0,05 mol) 2-Hydroxymethyl-1-1-{N-[3-methyl-4-(1,1,2,2-tetrafluorethoxy)-phenyl]-carbamoyl}-piperidin von Bsp. 2 in 100 ml Acetonitril und tropft bei 20 °C 3,4 g (0,06 mol) Methylisocyanat zu. Nach Zugabe von 50 mg Diazabicyclo[2.2.2]nonan rührt man 1 h bei 20 °C und 4 h bei 50 °C. Man destilliert das Lösungsmittel ab und verreibt den harzigen Rückstand mit einem Gemisch aus Ether/Hexan (1:1). Der kristalline Niederschlag wird abgesaugt und getrocknet. Man erhält 18,5 g (88 % d. Th.) 2-(N-Methyl-carbamoyloxymethyl)-1-{N-[3-methyl-4-(1,1,2,2-tetrafluorethoxy)-phenyl]-carbamoyl}-piperidin in Form farbloser Kristalle mit Schmp. 128 - 132 °C.

Beispiel 5

1-[N-(4-Chlor-2-fluor-5-methoxy-phenyl)-carbamoyl]-2-pivaloyloxy-methyl-piperidin

Man löst 15,9 g (0,05 mol) 1-[N-(4-Chlor-2-fluor-5-methoxy-phenyl)-carbamoyl]-2-hydroxymethylpiperidin (Bsp. 1) und 4,7 g (0,06 mol) Pyridin in 200 ml Toluol und tropft bei 20 °C 6,0 g (0.05 mol) Pivalinsäurechlorid zu. Man rührt 3 h bei 50 °C, wäscht zweimal mit je 100 ml Wasser und trocknet die Toluolphase über Natriumsulfat. Nach dem Abziehen des Lösungsmittels erhält man 18,4 g (92 % d. Th.) 1-[N-(4-Chlor-2-fluor-5-methoxy-phenyl)-carbamoyl]-2-pivaloyloxymethyl-piperidin in form eines farblosen zähen Öles.

Beispiel 6

1-[N-(4-Chlor-2-fluor-5-methoxy-phenyl)-carbamoyl]-2-ethoxycarbonyloxymethyl-piperidin

Man löst 15,9 g (0,05 mol) 1-[N-(4-Chlor-2-fluor-5-methoxy-phenyl)-carbamoyl]-2-hydroxymethyl-piperidin (Bsp. 1) und 6,0 g (0,06 mol) Triethylamin in 150 ml Toluol und tropft bei 20 °C 5,4 g (0,05 mol) Chlorameisensäureethylester zu. Man rührt 2 h bei 40 °C, wäscht zweimal mit je 50 ml Wasser und trocknet die Toluolphase über Natriumsulfat. Nach dem Abziehen des Lösungsmittels erhält man 19,0 g (97 % d. Th.) 1-[N-(4-Chlor-2-fluor-5-methoxy-phenyl)-carbamoyl]-2-ethoxycarbonyloxy-methyl-piperidin in Form eines zähen hellgelben Öles.

In analoger Weise, wie in den Beispielen 1 - 6 beschrieben, erhält man die Verbindungen der nachfolgenden Tabelle I.

Tabelle 1

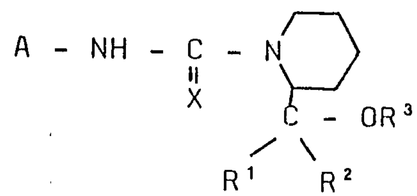

| Bsp. | $R^1$ | $R^2$ | $R^3$ | X | A | Fp [°C] |
|---|---|---|---|---|---|---|
| 7 | H | H | H | O | 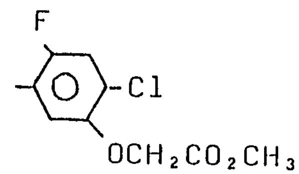 | Harz |
| 8 | H | H | H | O | 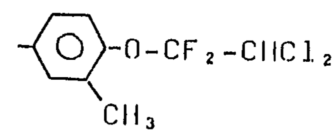 | |
| 9 | H | H | H | O | $3-CF_3-4-OC_2H_5-C_6H_3$ | 168-170 |
| 10 | H | H | H | O | $4-Cl-C_6H_4$ | 125-127 |
| 11 | H | H | H | O | $4-Br-C_6H_4$ | 132-138 |
| 12 | H | H | H | O | $4-i-C_3H_7-C_6H_4$ | Harz |
| | | | | | | ... |

Fortsetzung Tabelle 1

| Bsp. | $R^1$ | $R^2$ | $R^3$ | X | A | Fp [°C] |
|---|---|---|---|---|---|---|
| 13 | H | H | H | O | $3,4-Cl_2-C_6H_3$ | |
| 14 | H | H | H | O | $3-CH_3-4-Cl-C_6H_3$ | |
| 15 | H | H | H | O | $4-(4-Cl-C_6H_4O)-C_6H_4$ | |
| 16 | H | H | H | O | $2,4-Cl_2-5-(-CO_2C_2H_5)-C_6H_2$ | 137-141 |
| 17 | H | H | H | O | $2,4-F_2-C_6H_3$ | |
| 18 | H | H | H | O | $4-F-C_6H_4$ | |
| 19 | H | H | H | O | $3,5-Cl_2-C_6H_3$ | |
| 20 | H | H | H | O | $4-CH_3-3-OCH_3-C_6H_3$ | |
| 21 | H | H | H | O | $3-CF_3-C_6H_4$ | |
| 22 | H | H | H | O | $3-(HF_2C-CF_2-O)-C_6H_4$ | |
| 23 | H | H | H | O | $3-(ClFHC-CF_2-O)-C_6H_4$ | 102-107 |
| 24 | H | H | H | O | $3-(Cl_2HC-CF_2-O)-C_6H_4$ | |
| 25 | H | H | H | O | $4-(F_2HC-CF_2-O)-C_6H_4$ | 104-106 |
| 26 | H | H | H | O | $4-F_3CO-C_6H_4$ | |
| 27 | H | H | H | O | $4-(HC\equiv C-CH_2-O)-C_6H_4$ | |
| 28 | H | H | H | O | $4-(BrFHC-CF_2-O)-C_6H_4$ | |
| 29 | H | H | H | O | $4-(ClHC=CCl-O)-C_6H_4$ | |
| 30 | H | H | H | O | $4-(F_3C-CFH-CF_2-O)-C_6H_4$ | |

...

Fortsetzung Tabelle 1

| Bsp. | $R^1$ | $R^2$ | $R^3$ | X | A | Fp [°C] |
|---|---|---|---|---|---|---|
| 31 | $CH_3$ | $CH_3$ | H | O | $4-Cl-2-F-5-OCH_3-C_6H_2$ | |
| 32 | " | " | H | O | $4-Cl-C_6H_4$ | |
| 33 | " | " | H | O | $3-CF_3-4-C_2H_6O-C_6H_3$ | |
| 34 | " | " | H | O | $3-CH_3-4-(F_2H-CF_2-O)-C_6H_3$ | |
| 35 | H | H | H | S | $4-Cl-C_6H_4$ | |
| 36 | H | H | H | S | $3-Cl-4-CH_3-C_6H_3$ | |
| 37 | H | H | H | S | $3,4-Cl_2-C_6H_3$ | |
| 38 | H | H | H | S | $3-CF_3-C_6H_4$ | |
| 39 | H | H | H | S | $3-CF_3-4-C_2H_6O-C_6H_3$ | |
| 40 | H | H | H | S | $3-CH_3-4-(F_2HC-CF_2-O)-C_6H_3$ | |
| 41 | H | H | $-C(=O)-CH_3$ | O | $4-Cl-2-F-5-H_3CO-C_6H_2$ | |
| 42 | H | H | $-C(=O)-NH-CH_3$ | O | " | |
| 43 | H | H | $-C(=O)-CH_3$ | O | $3-CF_3-4-C_2H_5O-C_6H_3$ | |
| 44 | H | H | $-C(=O)-NHCH_3$ | O | $3-CH_3-4-(F_2HC-CF_2-O)$ $C_6H_3$ | |
| 45 | H | H | H | O | $3-Cl-5-F-2-OCH_3-C_6H_2$ | Harz |
| 46 | H | H | H | O | $2,4-Cl_2-6-F-3-OCH_3-C_6H$ | 136-142 |

Fortsetzung Tabelle 1

| Bsp. | $R_1$ | $R_2$ | $R_3$ | X | A | Fp [°C] |
|---|---|---|---|---|---|---|
| 47 | $CH_3$ | H | $CH_3$ | O | $4-Cl-2F-5-OCH_3-C_6H_2$ | Öl |
| 48 | $CH_3$ | $CH_3$ | H | O | $4-Cl-C_6H_4$ | Harz |
| 49 | $CH_3$ | $CH_3$ | H | O | $4-Cl-2-F-5-OCH_3-C_6H_2$ | Sirup |
| 50 | H | H | $2-Cl-4-CF_3-C_6H_3$ | O | $4-Cl-C_6H_4$ | Harz |
| 51 | H | $C_6H_5$ | H | O | $4-Cl-2-F-5-OCH_3-C_6H_2$ | 64-68 |
| 52 | H | H | $2-CF_3-C_6H_4$ | O | $4-Cl-C_6H_4$ | 137-142 |
| 53 | H | H | $CH(C_6H_5)_2$ | O | $4-Cl-2-F-5-OCH_3-C_6H_2$ | Harz |
| 54 | H | H | $C_2H_5$ | O | $4-Cl-2-F-5-OCH_3-C_6H_2$ | Sirup |
| 55 | H | $C_6H_5$ | $C_6H_5$ | O | $4-Cl-2-F-5-OCH_3-C_6H_5$ | Sirup |
| 56 | H | $CH_3$ | H | O | $4-Cl-2-F-5-OCH_3-C_6H_5$ | Öl |
| 57 | H | $C_2H_5$ | $4-CF_3-C_6H_4$ | O | $4-Cl-2-F-5-OCH_3-C_6H_5$ | Harz |
| 58 | H | H | H | O | $4-Cl-2-F-5-OCH_2C=CH-C_6H_2$ | Sirup |
| 59 | H | H | $C(O)C(CH_3)_3$ | O | $4-Cl-2-F-5-OCH_2C=CH-C_6H_2$ | Sirup |
| 60 | H | H | -H | O | $4-Cl-2-F-5-OCH_2C=CH-C_6H_2$ | Harz |
| 61 | H | H | $-CO-C(CH_3)_3$ | O | $4-Cl-2-F-5-OCH_2C=CH-C_6H_2$ | Harz |
| 62 | H | H | H | O | $4-Cl-2-F-5-[OCH(CH_3)CO_2C_2H_5]-C_6H_2$ | Sirup |
| 63 | H | H | $-CO-C(CH_3)_3$ | O | $4-Cl-2-F-5-[OCH(CH_3)CO_2C_2H_5]-C_6H_2$ | Sirup |
| 64 | H | H | $PS(OC_2H_5))_2$ | O | $4-Cl-2-F-5-OCH_3-C_6H_2$ | Harz |
| 65 | H | H | H | O | $4-Cl-2-F-5-OCH(CH_3)_2-C_6H_2$ | 144-149 |
| 66 | H | H | $CH_2-(4-Cl-C_6H_4)$ | O | $4-Cl-2-F-5-OCH_3-C_6H_2$ | 114-120 |
| 67 | H | H | H | O | $4-Br-2-F-4-OCH(CH_3)_2-C_6H_2$ | 144-149 |
| 68 | H | H | $C_2H_5$ | O | $4-Cl-2-F-5-OCH_3-C_6H_2$ | Öl |

Biologische Beispiele

Die Schädigung der Unkrautpflanzen bzw. die Kulturpflanzenverträglichkeit wurde gemäß einem Schlüssel bonitiert, in dem die Wirksamkeit durch Wertzahlen von 0 - 5 ausgedrückt ist.

Dabei bedeutet:

0 - ohne Wirkung bzw. Schaden
1 = 0 - 20 % Wirkung bzw. Schaden
2 = 20 - 40 % Wirkung bzw. Schaden
3 = 40 - 60 % Wirkung bzw. Schaden
4 = 60 - 80 % Wirkung bzw. Schaden
5 = 80 - 100 % Wirkung bzw. Schaden

1. Unkrautwirkung im Vorauflauf

Samen bzw. Rhizomstücke von mono- und dikotylen Unkrautpflanzen wurden in Plastiktöpfen (Ø = 9 cm) in sandiger Lehmerde ausgelegt und mit Erde abgedeckt. Die in Form von benetzbaren Pulvern oder Emulsionskonzentraten formulierten erfindungsgemäßen Verbindungen wurden dann als wäßrige Suspensionen bzw. Emulsionen mit einer Wasseraufwandmenge von umgerechnet 600 - 800 1/ha in unterschiedlichen Dosierungen auf die Oberfläche der Abdeckerde appliziert.

Nach der Behandlung wurden die Töpfe im Gewächshaus aufgestellt und unter guten Wachstumsbedingungen für die Unkrautpflanzen gehalten (Temperatur 23 plus/minus 1 °C, relative Luftfeuchte 60 - 80 %).

Die optische Bonitur der Pflanzen- bzw. Auflaufschäden erfolgte nach dem Auflaufen der Versuchspflanzen nach einer Versuchszeit von 3 - 4 Wochen im Vergleich zu unbehandelten Kontrollen.

Wie die Boniturwerte in Tabelle 2 zeigen, weisen die erfindungsgemäßen Verbindungen eine gute herbizide Vorauflaufwirksamkeit gegen ein breites Spektrum von Ungräsern und Unkräutern auf.

Tabelle 2: Vorauflaufwirkung

| Verbindung Bsp.-Nr. | Dosis kg a.i./ha | herbizide Wirkung SIA | CRS | ECG | LOM |
|---|---|---|---|---|---|
| 1 | 2,5 | 5 | 5 | 5 | 5 |
| 3 | 2,5 | 5 | 5 | 5 | 5 |
| 5 | 2,5 | 5 | 5 | 5 | 5 |
| 66 | 2,5 | 5 | 5 | 5 | 5 |
| 47 | 2,5 | 5 | 5 | 5 | 5 |
| 49 | 2,5 | 5 | 5 | 4 | 5 |
| 5 | 2,5 | 5 | 5 | 4 | 4 |

2. Unkrautwirkung in Nachauflauf

Samen bzw. Rhizomstücke von mono- und dikotylen Unkräutern wurden in Plastiktöpfen (Ø = 9 cm) in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt.

Die als Spritzpulver bzw. als Emulsionskonzentrate formulierten erfindungsgemäßen Verbindungen wurden in verschiedenen Dosierungen mit einer Wasseraufwandmenge von umgerechnet 600 l/ha auf die grünen Pflanzenteile gesprüht und nach ca. 3 - 4 Wochen Standzeit der Versuchspflanzen im Gewächshaus unter optimalen Wachstumsbedingungen (Temperatur 23 plus/minus 1 °C, relative Luftfeuchte 60 - 80 %) die Wirkung der Präparate optisch im Vergleich zu unbehandelten Kontrollen bonitiert.

Die erfindungsgemäßen Mittel weisen auch im Nachauflauf eine gute herbizide Wirksamkeit gegen ein breites Spektrum wirtschaftlich wichtiger Ungräser und Unkräuter auf. (Tabelle 3).

Tabelle 3: Nachauflaufwirkung

| Verbindung Bsp.-Nr. | Dosis kg a.i./ha | herbizide Wirkung SIA | ECG | LOM |
|---|---|---|---|---|
| 1 | 2,5 | 4 | 4 | 5 |
| 3 | 2,5 | 4 | 4 | 3 |
| 5 | 2,5 | 5 | 3 | 5 |
| 66 | 2,5 | 3 | 4 | 5 |
| 47 | 2,5 | 5 | - | 5 |
| 49 | 2,5 | 4 | - | 3 |

Abkürzungen:

SIA = Sinapis alba

CRS = Chrysanthemum segetum

ECG = Echinochloa crus-galli

LOM = Lolium multiflorum

**Patentansprüche**

1. Verbindungen der Formel I

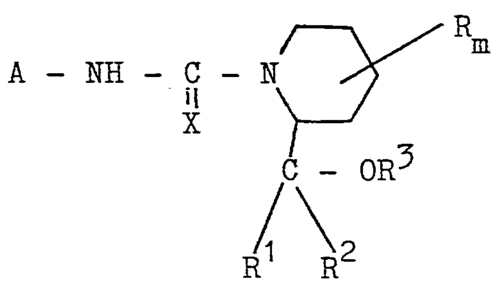

I,

worin

X Sauerstoff oder Schwefel;

R ($C_1$-$C_4$)Alkyl

$R^1$, $R^2$ unabhängig voneinander Wasserstoff, ($C_1$-$C_4$)Alkyl, Phenyl oder Benzyl, die beide bis zu dreifach durch ($C_1$-$C_4$)Alkyl, Halogen, $CF_3$, $NO_2$ oder CN substituiert sein können,

$R^3$ Wasserstoff, ($C_1$-$C_4$)Alkyl, das unsubstituiert oder bis zu dreifach substituiert ist durch Halogen, ($C_3$-$C_6$)Cycloalkyl, ($C_3$-$C_4$)Alkenyl, ($C_5$-$C_6$)Cycloalkenyl, ($C_3$-$C_4$)Alkinyl, ($C_1$-$C_4$)Alkoxy-($C_1$-$C_4$)alkyl oder ($C_1$-$C_4$)Alkoxycarbonyl-($C_1$-$C_4$)alkyl; Phenyl oder Benzyl, die beide unsubstituiert oder bis zu dreifach substituiert sind durch Halogen, ($C_1$-$C_4$)Alkyl, $NO_2$, $CF_3$, ($C_1$-$C_4$)Alkoxy oder ($C_1$-$C_4$)Alkoxycarbonyl; oder einen Rest der Formeln

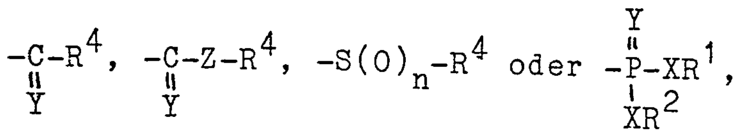

Y Sauerstoff oder Schwefel,
Z Sauerstoff, Schwefel oder $-NR^1$,
$R^4$ Wasserstoff, $(C_1-C_8)$Alkyl, das unsubstituiert oder bis zu dreifach substituiert ist durch Halogen, Phenyl oder Benzyl, die beide unsubstituiert oder bis zu dreifach am Aromaten substituiert sind durch Halogen, $(C_1-C_4)$Alkyl, $(C_1-C_4)$Alkoxy, $(C_1-C_4)$Alkoxycarbonyl, $NO_2$, $CF_3$ oder CN,
m 0, 1 oder 2,
n 1 oder 2,
A Phenyl oder Naphthyl, die beide unsubstituiert oder bis zu vierfach substituiert sind durch Halogen, $(C_1-C_4)$Alkyl, $(C_1-C_4)$Halogenalkyl, $(C_1-C_4)$Alkoxy, $(C_1-C_4)$Halogenalkoxy, $(C_3-C_6)$Cycloalkoxy, $(C_2-C_4)$Alkenyloxy, $(C_2-C_4)$Halogenalkenyloxy, $(C_5-C_6)$Cycloalkenyloxy, $(C_3-C_4)$Alkinyloxy, $(C_3-C_4)$Halogenalkinyloxy, $(C_1-C_4)$Alkoxy$(C_1-C_4)$alkoxy, $(C_1-C_4)$Alkoxycarbonyl, $(C_1-C_4)$Alkoxycarbonyl$(C_1-C_4)$alkoxy, $(C_1-C_4)$Alkoxycarbonyl-$(C_1-C_4)$alkoxycarbonyl, $(C_1-C_4)$Alkylthio, $(C_1-C_4)$Alkylsulfinyl, $(C_1-C_4)$Alkylsulfonyl, $(C_1-C_4)$Alkoxycarbonyl$(C_1-C_4)$alkylthio, Phenoxy, Benzyloxy, Phenoxymethyl, wobei die drei letztgenannten Reste im Phenylring bis zu zweifach durch Halogen, $CF_3$ oder $(C_1-C_4)$Alkyl substituiert sein können, $NO_2$, $CF_3$, CN oder Pyridyl, das unsubstituiert oder bis zu dreifach durch Halogen, $(C_1-C_4)$Alkyl, $NO_2$, $CF_3$ oder $(C_1-C_4)$Alkoxy substituiert ist,
bedeuten.

2. Verbindungen der Formel I, worin R = $(C_1-C_4)$Alkyl,
$R^1$ und $R^2$ unabhängig voneinander Wasserstoff, $(C_1-C_4)$Alkyl
$R^3$ Wasserstoff, $(C_1-C_4$—Alkyl, 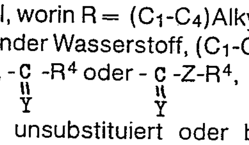,
$R^4$ $(C_1-C_4)$Alkyl, Phenyl, das unsubstituiert oder bis zu zweifach substituiert ist durch Halogen, $(C_1-C_4)$Alkyl oder $CF_3$,
A Phenyl, das bis zu dreifach durch Halogen, $(C_1-C_4)$Alkoxy, $(C_1-C_4)$Halogenalkoxy, $(C_1-C_4)$Alkylthio, $(C_1-C_4)$Alkylsulfonyl, Propargyloxy, $(C_1-C_4)$-Alkoxycarbonyl oder $(C_1-C_4)$Alkyloxycarbonyl$(C_1-C_4)$alkoxy, substituiert ist, und
m 0 oder 1
bedeuten.

3. Verfahren zur Herstellung von Verbindungen der Formel I gemäß Anspruch 1, dadurch gekennzeichnet, daß man

a) eine Verbindung der Formel II mit einem Piperidin-Derivat der Formel III

$$A - N = C = X \qquad (II)$$

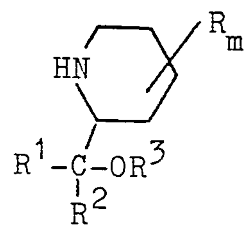
(III)

b) oder ein Amin der Formel IV mit einer Verbindung der Formel V

$A-NH_2$ +

(IV)

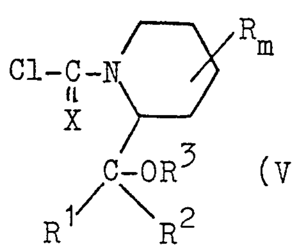
(V)

in Gegenwart einer Base umsetzt und die unter a) oder b) erhaltenen Verbindungen der Formel I für $R^3$ = H gegebenenfalls O-alkyliert oder O-acyliert.

4. Verwendung der Verbindungen der Formel I von Anspruch 1 oder 2 als Herbizide.

5. Herbizide Mittel, dadurch gekennzeichnet, daß sie eine Verbindung der Formel I von Anspruch 1 oder 2 enthalten.

6. Verfahren zur Bekämpfung von unerwünschten Pflanzen, dadurch gekennzeichnet, daß man eine wirksame Menge einer Verbindung der Formel I von Anspruch 1 oder 2 auf die zu behandelnden Anbauflächen oder die zu behandelnden Pflanzen aufbringt.

Patentansprüche für den folgenden Vertragsstaat: Österreich:

1. Verfahren zur Herstellung der Verbindung der Formel I

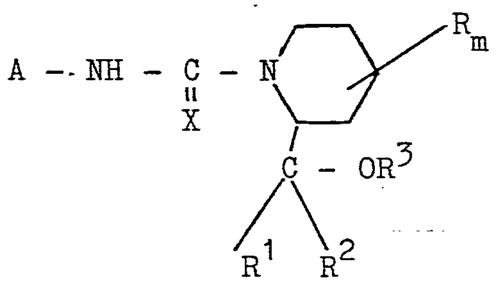

I,

worin
X Sauerstoff oder Schwefel;
R $(C_1$-$C_4)$Alkyl
$R^1$, $R^2$ unabhängig voneinander Wasserstoff, $(C_1$-$C_4)$Alkyl, Phenyl oder Benzyl, die beide bis zu dreifach durch $(C_1$-$C_4)$Alkyl, Halogen, $CF_3$, $NO_2$ oder CN substituiert sein können,
$R^3$ Wasserstoff, $(C_1$-$C_4)$Alkyl, das unsubstituiert oder bis zu dreifach substituiert ist durch Halogen, $(C_3$-$C_6)$Cycloalkyl, $(C_3$-$C_4)$Alkenyl, $(C_5$-$C_6)$Cycloalkenyl, $(C_3$-$C_4)$Alkinyl, $(C_1$-$C_4)$Alkoxy-$(C_1$-$C_4)$alkyl oder $(C_1$-$C_4)$Alkoxycarbonyl-$(C_1$-$C_4)$alkyl; Phenyl oder Benzyl, die beide unsubstituiert oder bis zu dreifach substituiert sind durch Halogen, $(C_1$-$C_4)$Alkyl, $NO_2$, $CF_3$, $(C_1$-$C_4)$Alkoxy oder $(C_1$-$C_4)$Alkoxycarbonyl; oder einen Rest der Formeln

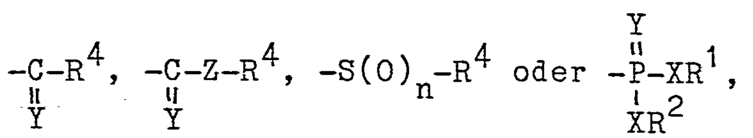

Y Sauerstoff oder Schwefel,
Z Sauerstoff, Schwefel oder $-NR^1$,
$R_4$ Wasserstoff, $(C_1$-$C_8)$Alkyl, das unsubstituiert oder bis zu dreifach substituiert ist durch Halogen, Phenyl oder Benzyl, die beide unsubstituiert oder bis zu dreifach am Aromaten substituiert sind durch Halogen, $(C_1$-$C_4)$Alkyl, $(C_1$-$C_4)$Alkoxy, $(C_1$-$C_4)$Alkoxycarbonyl, $NO_2$, $CF_3$ oder CN,
m 0, 1 oder 2,
n 1 oder 2,
A Phenyl oder Naphthyl, die beide unsubstituiert oder bis zu vierfach substituiert sind durch Halogen, $(C_1$-$C_4)$Alkyl, $(C_1$-$C_4)$Halogenalkyl, $(C_1$-$C_4)$Alkoxy, $(C_1$-$C_4)$Halogenalkoxy, $(C_3$-$C_6)$Cycloalkoxy, $(C_2$-$C_4)$Alkenyloxy, $(C_2$-$C_4)$Halogenalkenyloxy, $(C_5$-$C_6)$Cycloalkenyloxy, $(C_3$-$C_4)$Alkinyloxy, $(C_3$-$C_4)$Halogenalkinyloxy, $(C_1$-$C_4)$Alkoxy$(C_1$-$C_4)$alkoxy, $(C_1$-$C_4)$alkoxycarbonyl, $(C_1$-$C_4)$Alkoxycarbonyl$(C_1$-$C_4)$Alkoxy, $(C_1$-$C_4)$Alkoxycarbonyl-$(C_1$-$C_4)$alkoxycarbonyl, $(C_1$-$C_4)$Alkylthio, $(C_1$-$C_4)$Alkylsulfinyl, $(C_1$-$C_4)$Alkylsulfonyl, $(C_1$-$C_4)$Alkoxycarbonyl$(C_1$-$C_4)$alkylthio, Phenoxy, Benzyloxy, Phenoxymethyl, wobei die drei letztgenannten Reste im Phenylring bis zu zweifach durch Halogen, $CF_3$ oder $(C_1$-$C_4)$Alkyl substituiert sein können, $NO_2$, $CF_3$, CN oder Pyridyl, das unsubstituiert oder bis zu dreifach durch Halogen, $(C_1$-$C_4)$Alkyl, $NO_2$, $CF_3$ oder $(C_1$-$C_4)$Alkoxy substituiert ist,
bedeuten, dadurch gekennzeichnet, daß man

a) eine Verbindung der Formel II mit einem Piperidin-Derivat der Formel III

$$A - N = C = X \qquad (II)$$

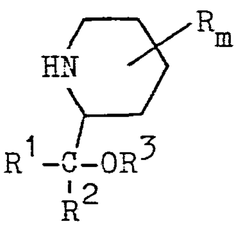

(III)

b) oder ein Amin der Formel IV mit einer Verbindung der Formel V

$A-NH_2$ + 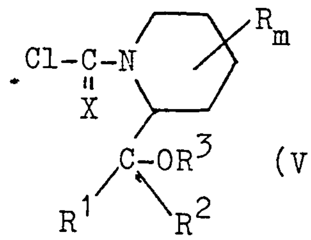

(IV) (V)

in Gegenwart einer Base umsetzt und die unter a) oder b) erhaltenen Verbindungen der Formel I für $R^3$ = H gegebenenfalls O-alkyliert oder O-acyliert.

2. Verfahren nach Anspruch 1, wobei in Formel I, II, III, IV und V bedeuten:
$R^1$ und $R^2$ unabhängig voneinander Wasserstoff, $(C_1\text{-}C_4)$Alkyl
$R^3$ Wasserstoff, $(C_1\text{-}C_4)$Alkyl, $-\underset{Y}{C}-R^4$ oder $-\underset{Y}{C}-Z-R^4$,
$R^4$ $(C_1\text{-}C_4)$Alkyl, Phenyl, das unsubstituiert oder bis zu zweifach substituiert ist durch Halogen, $(C_1\text{-}C_4)$Alkyl oder $CF_3$,
A Phenyl, das bis zu dreifach durch Halogen, $(C_1\text{-}C_4)$Alkoxy, $(C_1\text{-}C_4)$Halogenalkoxy, $(C_1\text{-}C_4)$Alkylthio, $(C_1\text{-}C_4)$Alkylsulfonyl, Propargyloxy, $(C_1\text{-}C_4)$Alkoxycarbonyl oder $(C_1\text{-}C_4)$Alkyloxycarbonyl$(C_1\text{-}C_4)$alkoxy, substituiert ist, und
m 0 oder 1.

3. Verfahren zur Bekämpfung von unerwünschten Pflanzen, dadurch gekennzeichnet, daß man eine wirksame Menge einer Verbindung der Formel I von Anspruch 1 oder 2 auf die zu behandelnden Anbauflächen oder die zu behandelnden Pflanzen aufbringt.

4. Herbizide Mittel, dadurch gekennzeichnet, daß sie eine Verbindung der Formel I von Anspruch 1 oder 2 enthalten.

5. Verwendung der Verbindungen der Formel I von Anspruch 1 oder 2 als Herbizide.

Patentansprüche für den folgenden Vertragsstaat: Spanien:

1. Verfahren zur Herstellung der Verbindung der Formel I

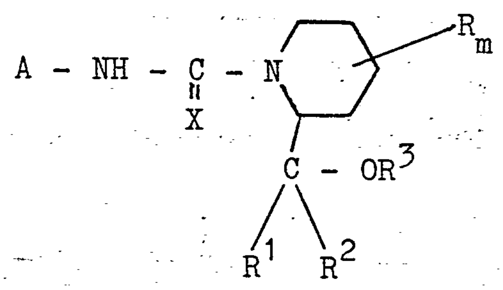

I,

worin
X Sauerstoff oder Schwefel;
R $(C_1\text{-}C_4)$Alkyl
$R^1$, $R^2$ unabhängig voneinander Wasserstoff, $(C_1\text{-}C_4)$Alkyl, Phenyl oder Benzyl, die beide bis zu dreifach durch $(C_1\text{-}C_4)$Alkyl, Halogen, $CF_3$, $NO_2$ oder CN substituiert sein können,
$R^3$ Wasserstoff, $(C_1\text{-}C_4)$Alkyl, das unsubstituiert oder bis zu dreifach substituiert ist durch Halogen, $(C_3\text{-}C_6)$Cycloalkyl, $(C_3\text{-}C_4)$Alkenyl, $(C_5\text{-}C_6)$Cycloalkenyl, $(C_3\text{-}C_4)$Alkinyl, $(C_1\text{-}C_4)$Alkoxy-$(C_1\text{-}C_4)$alkyl oder $(C_1\text{-}C_4)$Alkoxycarbonyl-$(C_1\text{-}C_4)$alkyl; Phenyl oder Benzyl, die beide unsubstituiert oder bis zu dreifach substituiert sind durch Halogen, $(C_1\text{-}C_4)$Alkyl, $NO_2$, $CF_3$, $(C_1\text{-}C_4)$Alkoxy oder $(C_1\text{-}C_4)$Alkoxycarbonyl; oder einen Rest der Formeln

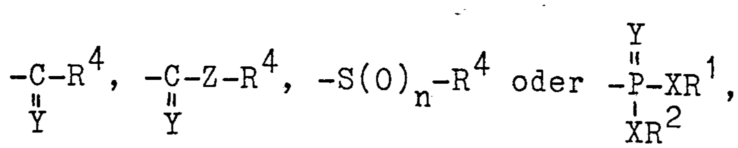

Y Sauerstoff oder Schwefel,
Z Sauerstoff, Schwefel oder $-NR^1$,
$R^4$ Wasserstoff, $(C_1\text{-}C_8)$Alkyl, das unsubstituiert oder bis zu dreifach substituiert ist durch Halogen, Phenyl oder Benzyl, die beide unsubstituiert oder bis zu dreifach am Aromaten substituiert sind durch Halogen, $(C_1\text{-}C_4)$Alkyl, $(C_1\text{-}C_4)$Alkoxy, $(C_1\text{-}C_4)$Alkoxycarbonyl, $NO_2$, $CF_3$ oder CN,

m 0, 1 oder 2,
n 1 oder 2,
A Phenyl oder Naphthyl, die beide unsubstituiert oder bis zu vierfach substituiert sind durch Halogen, $(C_1$-$C_4)$Alkyl, $(C_1$-$C_4)$Halogenalkyl, $(C_1$-$C_4)$Alkoxy, $(C_1$-$C_4)$Halogenalkoxy, $(C_3$-$C_6)$Cycloalkoxy, $(C_2$-$C_4)$Alkenyloxy, $(C_2$-$C_4)$Halogenalkenyloxy, $(C_5$-$C_6)$Cycloalkenyloxy, $(C_3$-$C_4)$Alkinyloxy, $(C_3$-$C_4)$Halogenalkinyloxy, $(C_1$-$C_4)$Alkoxy$(C_1$-$C_4)$alkoxy, $(C_1$-$C_4)$Alkoxycarbonyl, $(C_1$-$C_4)$Alkoxycarbonyl$(C_1$-$C_4)$alkoxy, $(C_1$-$C_4)$Alkoxycarbonyl-$(C_1$-$C_4)$alkoxycarbonyl, $(C_1$-$C_4)$Alkylthio, $(C_1$-$C_4)$Alkylsulfinyl, $(C_1$-$C_4)$Alkylsulfonyl, $(C_1$-$C_4)$Alkoxycarbonyl$(C_1$-$C_4)$alkylthio, Phenoxy, Benzyloxy, Phenoxymethyl, wobei die drei letztgenannten Reste im Phenylring bis zu zweifach durch Halogen, $CF_3$ oder $(C_1$-$C_4)$Alkyl substituiert sein können, $NO_2$, $CF_3$, CN oder Pyridyl, das unsubstituiert oder bis zu dreifach durch Halogen, $(C_1$-$C_4)$Alkyl, $NO_2$, $CF_3$ oder $(C_1$-$C_4)$Alkoxy substituiert ist,
bedeuten, dadurch gekennzeichnet, daß man

a) eine Verbindung der Formel II mit einem Piperidin-Derivat der Formel III

$A - N = C = X$ (II)

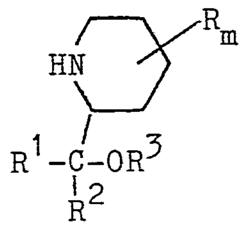

(III)

b) oder ein Amin der Formel IV mit einer Verbindung der Formel V

$A-NH_2$ +

(IV)

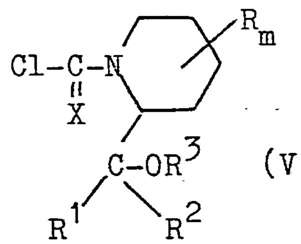

(V)

in Gegenwart einer Base umsetzt und die unter a) oder b) erhaltenen Verbindungen der Formel I für $R^3 = H$ gegebenenfalls O-alkyliert oder O-acyliert.

2. Verfahren nach Anspruch 1, wobei in Formel I, II, III, IV und V bedeuten:
$R^1$ und $R^2$ unabhängig voneinander Wasserstoff, $(C_1$-$C_4)$Alkyl
$R^3$ Wasserstoff, $(C_1$-$C_4)$Alkyl, $-\underset{\underset{Y}{\|}}{C}-R^4$ oder $-\underset{\underset{Y}{\|}}{C}-Z-R^4$,
$R^4$ $(C_1$-$C_4)$Alkyl, Phenyl, das unsubstituiert oder bis zu zweifach substituiert ist durch Halogen, $(C_1$-$C_4)$Alkyl oder $CF_3$,
A Phenyl, das bis zu dreifach durch Halogen, $(C_1$-$C_4)$Alkoxy, $(C_1$-$C_4)$Halogenalkoxy, $(C_1$-$C_4)$Alkylthio, $(C_1$-$C_4)$Alkylsulfonyl, Propargyloxy, $(C_1$-$C_4)$Alkoxycarbonyl oder $(C_1$-$C_4)$Alkyloxycarbonyl$(C_1$-$C_4)$alkoxy, substituiert ist, und
m 0 oder 1
bedeuten.

3. Verfahren zur Bekämpfung von unerwünschten Pflanzen, dadurch gekennzeichnet, daß man eine wirksame Menge einer Verbindung der Formel I von Anspruch 1 oder 2 auf die zu behandelnden Anbauflächen oder die zu behandelnden Pflanzen aufbringt.